# EUROPÄISCHE PATENTSCHRIFT

(11) **EP 3 856 070 B1**
(45) Veröffentlichungstag und Bekanntmachung des Hinweises auf die Patenterteilung: **01.03.2023**
(21) Anmeldenummer: 19773435.3
(22) Anmeldetag: 25.09.2019
(51) Int. Cl.: A61C 1/00

(54) **MEDIZINISCHES ODER DENTALES, MIT EINER HAND HALTBARES BEHANDLUNGSINSTRUMENT MIT EINEM KAPAZITIVEN SENSOR**
MEDICAL OR DENTAL TREATMENT INSTRUMENT WHICH CAN BE HELD IN A HAND COMPRISING A CAPACITIVE SENSOR
INSTRUMENT DE TRAITEMENT MÉDICAL OU DENTAIRE POUVANT ÊTRE TENU À LA MAIN DOTÉ D'UN CAPTEUR CAPACITIF

(30) Priorität: 25.09.2018 EP 18196500
(43) Veröffentlichungstag der Anmeldung: 04.08.2021
(73) Patentinhaber: W & H Dentalwerk Bürmoos GmbH, 5111 Bürmoos (AT)
(72) Erfinder: PRUCKNER, Christian, 1190 Wien (AT); SCHWARZ, Tobias, 5071 Wals (AT)
(74) Vertreter: Benda, Ralf
(86) Internationale Anmeldenummer: PCT/EP2019/075778
(87) Internationale Veröffentlichungsnummer: WO 2020/064785

(56) Entgegenhaltungen:
- EP-A1- 2 078 508
- WO-A1-80/01643
- WO-A1-2012/042493
- WO-A1-2015/073370

## Beschreibung

Die vorliegende Erfindung betrifft ein medizinisches oder dentales, mit einer Hand haltbares Behandlungsinstrument mit einem an einer Außenhülse vorgesehenen Stellelement mit einem kapazitiven Sensor.

Ein derartiges medizinisches oder dentales Behandlungsinstrument ist aus der WO 80/01643 A1 bekannt.

WO 2012/042493 A1 und WO 2015/073370 A1 offenbaren ein medizinisches oder dentales, mit einer Hand haltbares Behandlungsinstrument, umfassend: eine ein- oder mehrteilige Außenhülse und zumindest ein an der Außenhülse vorgesehenes Stellelement mit zumindest einem kapazitiven Sensor und einer an der Außenhülse vorgesehenen und operativ mit dem kapazitiven Sensor verbundenen Sensorfläche, bei deren Berührung oder bei einer Annäherung daran das Stellelement ein Stellsignal generiert, wobei eine Anzeigevorrichtung an dem mit einer Hand haltbaren Behandlungsinstrument angeordnet ist.

EP 2 078 508 A1 offenbart ein medizinisches oder dentales, mit einer Hand haltbares Behandlungsinstrument, umfassend: eine ein- oder mehrteilige Außenhülse und zumindest ein an der Außenhülse vorgesehenes Stellelement, wobei eine Anzeigevorrichtung mit einem Schwingungserzeuger, der Schwingungen in Form von Körperschall erzeugt, wobei die Anzeigevorrichtung an dem mit einer Hand haltbaren Behandlungsinstrument angeordnet ist und die Generierung eines Stellsignals die Erzeugung der Körperschallschwingungen und Übertragung der Körperschallschwingungen auf eine Anzeigefläche der Anzeigevorrichtung und/ oder die Sensorfläche anzeigt.

Ein Behandlungsinstrument mit einem an einer Außenhülse vorgesehenen Stellelement mit einem kapazitiven Sensor hat unter anderem den Vorteil, dass kein Bedienknopf, Schalter oder Taster an der Oberfläche der Außenhülse benötigt wird. Damit entfällt zum Beispiel die Notwendigkeit, eine Öffnung in der Außenhülse, in welcher ein derartiger Bedienknopf angeordnet ist, abzudichten.

Das Fehlen eines derartigen Bedienknopfs birgt jedoch in nachteiliger Weise die Gefahr, das Stellelement mit einem kapazitiven Sensor unbeabsichtigt und/ oder unbemerkt zu betätigen und damit einen unerwünschten, gegebenenfalls den Anwender oder Patienten gefährdenden Stellvorgang auszulösen. Diese Gefahr ist besonders groß, wenn das Stellelement mit einem kapazitiven Sensor an dem mit einer Hand haltbaren Behandlungsinstrument vorgesehen ist.

Der vorliegenden Erfindung liegt daher die Aufgabe zugrunde, ein medizinisches oder dentales, mit einer Hand haltbares Behandlungsinstrument mit einem an einer Außenhülse vorgesehenen Stellelement mit einem kapazitiven Sensor zu schaffen, das die im Vorstehenden genannten Nachteile nicht aufweist. Insbesondere soll eine unbeabsichtigte und/ oder unbemerkte Betätigung des Stellelements mit einem kapazitiven Sensor oder eine unbeabsichtigte und/ oder unbemerkte Erzeugung eines Stellsignals verhindert werden.

Diese Aufgabe wird gemäß der vorliegenden Erfindung durch ein medizinisches oder dentales, mit einer Hand haltbares Behandlungsinstrument mit den Merkmalen des Anspruchs 1 gelöst. Besonders vorteilhafte Ausführungsformen sind in den Unteransprüchen angeführt.

Das medizinische oder dentale, mit einer Hand haltbare Behandlungsinstrument, umfasst eine ein- oder mehrteilige Außenhülse und zumindest ein an oder in der Außenhülse vorgesehenes Stellelement mit zumindest einem kapazitiven Sensor und einer an der Außenhülse vorgesehenen und operativ mit dem kapazitiven Sensor verbundenen Sensorfläche, bei deren Berührung oder bei einer Annäherung daran das Stellelement ein Stellsignal generiert. Das Behandlungsinstrument umfasst des Weiteren eine Anzeigevorrichtung, die an (oder in) dem mit einer Hand haltbaren Behandlungsinstrument angeordnet ist. Die Anzeigevorrichtung ist ausgebildet,
(1) die operativ mit dem kapazitiven Sensor verbundene Sensorfläche anzuzeigen, oder
(2) die Generierung eines Stellsignals aufgrund einer Berührung der Sensorfläche oder Annäherung an die Sensorfläche anzuzeigen, oder
(3) die operativ mit dem kapazitiven Sensor verbundene Sensorfläche und die Generierung eines Stellsignals nach einer Berührung der Sensorfläche oder Annäherung an die Sensorfläche anzuzeigen.

Durch das Vorsehen der Anzeigevorrichtung wird in vorteilhafter Weise die Gefahr einer unbeabsichtigten und/ oder unbemerkten Betätigung des Stellelements mit einem kapazitiven Sensor oder einer unbeabsichtigten und/ oder unbemerkten Erzeugung eines Stellsignals erheblich verringert. Wie im Weiteren noch im Detail beschrieben ist, ist die Anzeigevorrichtung bevorzugt ausgebildet, die Sensorfläche und/ oder die Generierung eines Stellsignals optisch, akustisch und/ oder haptisch anzuzeigen oder ein optisches, akustisches und/ oder haptisches Anzeigesignal zu erzeugen und abzugeben.

Das medizinische oder dentale, mit einer Hand haltbare Behandlungsinstrument umfasst insbesondere zumindest eines der folgenden Elemente: ein gerades oder gebogenes Handstück; ein Winkelstück, an dem ein Werkzeug gewinkelt zu einer Längsachse eines Griffabschnitts befestigbar ist; ein Handgriffelement; ein mit einer Hand haltbares Behandlungsinstrument, welches vorgesehen ist, elektromagnetische Strahlung auf eine Behandlungsstelle abzugeben; ein mit einer Hand haltbares Behandlungsinstrument, welches vorgesehen ist, ein Fluid auf eine Behandlungsstelle abzugeben; ein mit einer Hand haltbares Behandlungsinstrument, welches vorgesehen ist, ein diagnostisches und/ oder therapeutisches Medium auf eine Behandlungsstelle abzugeben; ein mit einer Hand haltbares Behandlungsinstrument, welches vorgesehen ist, Schwingungen, insbesondere Ultraschallschwingungen, auf eine Behandlungsstelle abzugeben; eine Motoreinheit zum Antrieb eines auf eine Behandlungsstelle einwirkenden Behandlungswerkzeugs, insbesondere mit einem pneumatisch oder elektrisch betriebenen Motor; ein mit einer Hand haltbares Behandlungsinstrument mit einer Bildaufnahmevorrichtung, insbesondere mit einer Kamera; ein Kupplungselement zur Verbindung mit einer Medienquelle, insbesondere einer Flüssigkeitsquelle, einer Gasquelle oder einer Quelle von elektrischer Energie; eine Adaptereinheit.

Die ein- oder mehrteilige Außenhülse ist zum Beispiel aus Metall oder aus Kunststoff gefertigt. Die ein- oder mehrteilige Außenhülse ist vorzugsweise rohrförmig ausgebildet und/ oder weist einen hohlen Innenraum auf, insbesondere zur Aufnahme einer oder mehrerer Bauteile, zum Beispiel von Teilen der Anzeigevorrichtung. Die ein- oder mehrteilige Außenhülse umfasst (entlang ihrer Längsachse) vorzugsweise zumindest zwei gewinkelt (mit einem Winkel >0°) oder gebogen zueinander angeordnete Abschnitte.

Das an der Außenhülse vorgesehenes Stellelement mit zumindest einem kapazitiven Sensor ist vorzugsweise ausgebildet ein Stellglied ein- und auszuschalten. Das Stellglied weist insbesondere zumindest eines der folgenden Elemente auf: eine elektromagnetische Strahlungsquelle, insbesondere eine elektromagnetische Strahlungsquelle, die für den Menschen sichtbares Licht abgibt und/ oder die elektromagnetische Strahlung zur Erkennung einer Anomalie an Gewebe abgibt, zum Beispiel von Karies, Plaque und/ oder Zahnbelag; einen motorischen Antrieb, insbesondere einen Elektromotor, einen Luftmotor oder einen Schwingungen erzeugenden Motor, zum Beispiel einen Piezomotor oder einen magnetostriktiven Motor; eine Förder- oder Leitvorrichtung, zum Beispiel eine Pumpe oder ein Ventil, für ein Medium, insbesondere ein medizinisches, therapeutisches oder diagnostisches Behandlungsmedium und/ oder ein Kühlmedium, zum Beispiel Luft, Wasser, ein pulverförmiges Medium, ein Desinfektionsmittel, ein Anästhetikum oder ein Arzneimittel; eine Bildaufnahmevorrichtung, insbesondere ein Bildsensor, ein CMOS- oder CCD-Sensor oder eine Kamera; einen Sensor zur Detektion eines Messwertes, zum Beispiel einen Temperatursensor, einen Drucksensor, einen Leitfähigkeitssensor oder einen Feuchtigkeitssensor; ein Heizelement, insbesondere zum Erwärmen eines medizinischen, therapeutischen oder diagnostischen Behandlungsmediums.

Alternativ oder zusätzlich ist das an der Außenhülse vorgesehene Stellelement mit zumindest einem kapazitiven Sensor vorzugsweise ausgebildet einen Stellwert eines Stellglieds stufenlos oder in Stufen zu stellen. Das Stellglied umfasst vorzugsweise zumindest eines der im Vorstehenden genannten Stellglieder. Der Stellwert umfasst zum Beispiel zumindest einen der folgenden Parameter: eine Leistung, zum Beispiel eines motorischen Antriebs, einer Pumpe oder eines Heizelements; eine Intensität oder Pulsrate von emittierter Strahlung einer elektromagnetische Strahlungsquelle; eine Frequenz, zum Beispiel von erzeugten und/ oder emittierten Schwingungen; einen Volumenstrom, zum Beispiel eines medizinischen, therapeutischen oder diagnostischen Behandlungsmediums und/ oder eines Kühlmediums; eine Brennweite oder ein Vergrößerungsfaktor einer optischen Vorrichtung, insbesondere einer Kamera; eine Temperatur, zum Beispiel eines Heizelements.

Der zumindest eine kapazitive Sensor ist in bekannter Weise ausgebildet, auf Basis der Veränderung der elektrischen Kapazität eines oder mehrere Kondensatoren und/ oder des Behandlungsinstruments zu arbeiten, insbesondere ein Stellsignal zu generieren. Vorzugsweise weist der zumindest eine kapazitive Sensor starre und/ oder unbewegliche Platten auf, deren Kapazität sich aufgrund einer Annäherung oder einer Berührung durch ein Bedienelement ändert. Das Bedienelement umfasst zum Beispiel ein elektrisch leitendes Material oder ein Dielektrikum, insbesondere eine Hand oder einen Finger eines Anwenders.

Die Sensorfläche, bei deren Berührung oder bei einer Annäherung daran sich die Kapazität ändert und der zumindest eine kapazitive Sensor ein Stellsignal generiert, ist als Teil des zumindest einen kapazitiven Sensors ausgebildet und/ oder operativ mit diesem verbunden. Die Sensorfläche ist insbesondere integral oder einstückig mit der Außenhülse ausgebildet. Die Sensorfläche ist insbesondere durch einen Teil oder Abschnitt der ein- oder mehrteiligen Außenhülse gebildet. Damit werden in vorteilhafter Weise Schmutzkanten an der Oberfläche der Außenhülse und ein Abdichten der Sensorfläche vermieden.

Alternativ ist es auch möglich, die Sensorfläche als separates Bauteil auszubilden, welches, insbesondere bündig, in die ein- oder mehrteilige Außenhülse integriert ist. Die Sensorfläche umfasst dann zum Beispiel eine Glasfläche, eine Kunststofffläche, eine keramische Fläche und/ oder eine Metallfläche oder Metallfolie. Vorzugsweise ist in der Außenhülse eine Öffnung vorgesehen, in welcher die Sensorfläche aufgenommen ist. Vorzugsweise ist ein Dichtelement vorgesehen, um die Öffnung, in welcher die Sensorfläche aufgenommen ist, abzudichten.

Besonders bevorzugt ist nur ein Teil oder Abschnitt der Außenhülse als Stellelement und/ oder Sensorfläche ausgebildet. Insbesondere umfasst/ umfassen das Stellelement und/ oder die Sensorfläche eine geringere Fläche als die Gesamtfläche der Außenhülse des Behandlungsinstruments. Damit ist in vorteilhafter Weise die Gefahr einer unbeabsichtigten und/ oder unbemerkten Betätigung des Stellelements weiter reduziert.

Vorzugsweise ist eine elektrische Versorgungsleitung vorgesehen, um den zumindest einen kapazitiven Sensor mit einer elektrischen Energiequelle zu verbinden und/ oder mit elektrischer Energie zu versorgen.

Vorzugsweise ist eine, insbesondere elektrische, Signalleitung vorgesehen, welche den zumindest einen kapazitiven Sensor und eine Steuerelektronik, die ausgebildet ist, das Stellsignal des kapazitiven Sensors zu empfangen und auszuwerten, verbindet. Insbesondere ist die Signalleitung ausgebildet, das Stellsignal des zumindest einen kapazitiven Sensors an die Steuerelektronik zu übertragen.

Vorzugsweise ist die elektrische Versorgungsleitung und/ oder Signalleitung zusätzlich zur Übertragung elektrischer Energie an einen in dem Behandlungsinstrument vorgesehenen oder damit verbundenen elektrischen Verbraucher, zum Beispiel eine elektromagnetische Strahlungsquelle, ausgebildet. Damit wird in vorteilhafter Weise eine Reduktion der elektrischen Leitungen des Behandlungsinstruments erreicht.

Vorzugsweise bilden der zumindest eine kapazitive Sensor, die Sensorfläche, die elektrische Versorgungsleitung, die Signalleitung und die Steuerelektronik einen elektrischen Schaltkreis zum Stellen oder Schalten eines Schaltglieds.

Vorzugsweise bedeckt oder bildet zumindest ein Teil der Anzeigevorrichtung, insbesondere eine Markierung oder eine Anzeigefläche der Anzeigevorrichtung, die ein Anzeigesignal emittiert, einen Teil der Sensorfläche. Alternativ bedeckt oder bildet zumindest ein Teil der Anzeigevorrichtung, insbesondere eine Markierung oder eine Anzeigefläche der Anzeigevorrichtung, die ein Anzeigesignal emittiert, die gesamte Sensorfläche. Vorzugsweise ist zumindest ein Teil der Anzeigevorrichtung, insbesondere eine Markierung oder eine Anzeigefläche der Anzeigevorrichtung, die ein Anzeigesignal emittiert, auf einem Teil der Sensorfläche oder der gesamten Sensorfläche angeordnet. Damit ist in vorteilhafter Weise die Position der Sensorfläche und/ oder deren Betätigung unmittelbar an der Sensorfläche selbst und/ oder durch die Sensorfläche angezeigt.

Besonders bevorzugt sind die Sensorfläche und eine Markierung oder eine Anzeigefläche der Anzeigevorrichtung, die ein Anzeigesignal emittiert, insbesondere eine Lichtabgabefläche, gleich groß und/ oder ident, i.e. durch dasselbe Bauteil gebildet. Besonders bevorzugt bildet eine transparente Kunststoff- oder Glasfläche oder eine metallische Fläche die idente Sensorfläche und Markierung oder Anzeigefläche. Vorzugsweise umfasst die Anzeigefläche einen Lichtleiter, der zumindest einen Teil der Sensorfläche bildet. Dies vereinfacht in vorteilhafter Weise die Herstellung des Behandlungsinstruments, da für die Sensorfläche und Markierung oder Anzeigefläche ein gemeinsames Bauteil ausreicht.

Alternativ sind die Sensorfläche und eine Anzeigefläche der Anzeigevorrichtung, die ein Anzeigesignal emittiert, insbesondere eine Lichtabgabefläche, voneinander getrennt und/ oder beabstandet und/ oder bilden zwei separate Flächen an der Oberfläche der Außenhülse des Behandlungsinstruments. Insbesondere umgibt zumindest ein Teil der Anzeigevorrichtung die Sensorfläche, vorzugsweise unmittelbar angrenzend. Zum Beispiel ist die Anzeigefläche, insbesondere einer Licht emittierende Anzeigevorrichtung, ringförmig um die Sensorfläche angeordnet. Damit ist in vorteilhafter Weise die Wahrnehmbarkeit der Anzeigevorrichtung, insbesondere einer Licht emittierenden Anzeigevorrichtung, verbessert.

Vorzugsweise ist eine Anzeigefläche der Anzeigevorrichtung, die ein Anzeigesignal emittiert, insbesondere eine Lichtabgabefläche oder eine Schwingungen abgebende Fläche, größere als die Sensorfläche. Bevorzugt ist die Sensorfläche als Teil dieser größeren Anzeigefläche ausgebildet. Damit ist in vorteilhafter Weise die Wahrnehmbarkeit der Anzeigevorrichtung verbessert.

Vorzugsweise bedeckt oder umfasst die Anzeigevorrichtung nur einen Teil der Außenhülse des mit einer Hand haltbaren Behandlungsinstruments.

Vorzugsweise ist zumindest ein Teil der Anzeigevorrichtung mit zumindest einem Abschnitt der Außenhülse, vorzugsweise mit zumindest einem Abschnitt der Sensorfläche operativ verbunden, insbesondere signalübertragend verbunden, schwingungsübertragend oder lichtleitend, zum Beispiel über einen Schwingstab oder einen Lichtleiter. Damit ist es in vorteilhafter Weise möglich, das von der Anzeigevorrichtung generierte Anzeigesignal gezielt an die Sensorfläche übertragen.

Vorzugsweise ist/ sind das Stellelement und/ oder der zumindest eine kapazitive Sensor und/ oder die Anzeigevorrichtung nur an einem Abschnitt der Außenhülse des Behandlungsinstruments ausgebildet, insbesondere an einem Kupplungs- oder Verbindungsabschnitt zur Verbindung mit einer Medien-, Energiequelle und/ oder Steuer- oder Regelvorrichtung oder an einem Zubehörhalteabschnitt, an dem ein Zubehör, zum Beispiel ein Werkzeug, ein Lichtleiter oder eine Düse, befestigbar ist. Damit wird in vorteilhafter Weise die Gefahr einer unbeabsichtigten und/ oder unbemerkten Betätigung des Stellelements weiter reduziert.

Vorzugsweise weist die Anzeigevorrichtung eine an der Oberfläche der Außenhülse und/ oder des Stellelements und/ oder der Sensorfläche für den Anwender sichtbare und/ oder fühlbare Markierung auf. Vorzugsweise ist die Markierung flächig ausgebildet oder umgibt die Sensorfläche rahmenförmig. Damit erkennt der Anwender in vorteilhafter Weise unmittelbar die Position des Stellelements und/ oder der Sensorfläche.

Vorzugsweise umfasst die Markierung eine Farbmarkierung. Besonders bevorzugt ist die Farbmarkierung auf der ein- oder mehrteiligen Außenhülse des Behandlungsinstruments vorgesehen, insbesondere auf deren (für den Anwender sichtbaren) Oberfläche oder Außenseite. Insbesondere unterscheidet sich die Farbe der Farbmarkierung von der Farbe der Außenhülse. Damit ist die Markierung für den Anwender in vorteilhafter Weise besonders gut erkennbar.

Vorzugsweise umfasst die Farbmarkierung ein an der Außenhülse befestigbares, insbesondere anhaftendes oder klebendes, Etikett. Alternativ umfasst die Farbmarkierung einen auf der Außenhülse auftragbaren Farbanstrich oder Lack. Alternativ umfasst die Farbmarkierung eine durch einen chemischen Prozess, zum Beispiel durch Oxidation, Eloxieren, Verzinken oder Nitrieren, bewirkte Farbänderung der ein- oder mehrteiligen Außenhülse des Behandlungsinstruments, insbesondere deren Oberfläche oder Außenseite. Alternativ umfasst die Farbmarkierung eine durch Belasern bewirkte Farbänderung der ein- oder mehrteiligen, insbesondere metallischen, Außenhülse des Behandlungsinstruments, insbesondere deren Oberfläche oder Außenseite. Alternativ umfasst die Farbmarkierung ein farbiges Fenster, zum Beispiel aus Glas oder Kunststoff, das in einer Öffnung der Außenhülse des Behandlungsinstruments aufgenommen ist und vorzugsweise auch als Sensorfläche ausgebildet ist. Alternativ umfasst die Farbmarkierung Farbpigmente, die in ein Material der Außenhülse, zum Beispiel Kunststoff, eingefügt und/ oder darin aufgenommen sind.

Vorzugsweise umfasst die Markierung eine für einen Anwender haptisch wahrnehmbare Markierung, die zur haptischen Wahrnehmung zumindest eine Vertiefung, Rille, Nut, einen Fortsatz, Vorsprung und/ oder eine Erhebung aufweist. Die haptisch wahrnehmbare Markierung ist zum Beispiel durch mechanische Bearbeitung und/ oder durch Erodieren in der Oberfläche der Außenhülse und/ oder des Stellelements und/ oder der Sensorfläche gebildet. Die haptisch wahrnehmbare Markierung umfasst zum Beispiel eine Riffelung. Eine haptisch wahrnehmbare Markierung ist in vorteilhafter Weise für den Anwender wahrnehmbar, ohne dass dieser auf die Markierung blicken muss.

Vorzugsweise ist die Markierung auf der Außenhülse befestigt oder aufgetragen oder als Teil der Außenhülse oder durch die Außenhülse oder an oder in der Außenhülse gebildet.

Die Anzeigevorrichtung zur Erzeugung von Schwingungen ist derart ausgebildet, um damit die Generierung eines Stellsignals aufgrund einer Berührung der Sensorfläche oder Annäherung an die Sensorfläche anzuzeigen. In vorteilhafter Weise ist es für den Anwender somit nicht notwendig zur Wahrnehmung eines von der Anzeigevorrichtung abgegebenen Signals auf die Anzeigevorrichtung blicken zu müssen.

Die Anzeigevorrichtung zur Erzeugung von Schwingungen umfasst einen Schwingungserzeuger, zum Beispiel zumindest ein Piezoelement, insbesondere einen Piezostapel, und/ oder einen, insbesondere elektrischen, Unwuchtmotor. Der Unwuchtmotor umfasst zur Erzeugung der Schwingungen insbesondere eine exzentrisch zu einer Rotationsachse einer Rotorwelle angeordnete Masse.

Die Anzeigevorrichtung zur Erzeugung von Schwingungen ist ausgebildet, die vom Schwingungserzeuger erzeugten Schwingungen in Form von Körperschall auf eine Anzeigefläche der Anzeigevorrichtung, die ein Anzeigesignal emittiert, und/ oder die Sensorfläche zu übertragen. Die Übertragung der Schwingungen auf die Anzeigefläche oder Sensorfläche erfolgt insbesondere direkt durch den Schwingungserzeuger selbst und/ oder über zumindest ein weiteres Bauteil der Anzeigevorrichtung, zum Beispiel einen Schwingstab, und/ oder über zumindest ein Bauteil des Behandlungsinstruments, zum Beispiel die Außenhülse, eine Platine, eine Medienleitung, ein Lager oder eine Welle. Damit ist das Anzeigesignal in vorteilhafter Weise für den Anwender vorwiegend haptisch wahrnehmbar und somit insbesondere in einer Umgebung mit vielen akustischen Signalen gut unterscheidbar.

Alternativ oder zusätzlich ist die Anzeigevorrichtung zur Erzeugung von für einen Anwender akustisch wahrnehmbaren Schwingungen ausgebildet. Die Anzeigevorrichtung weist vorzugsweise zumindest ein in Schwingung versetzbares Bauteil auf, zum Beispiel den Schwingungserzeuger selbst oder einen damit verbundenen Schwingstab, das Schwingungen an die Luft und/ oder an ein Bauteil des Behandlungsinstruments, zum Beispiel die Außenhülse, die Sensorfläche, das Stellelement, eine Medienleitung, ein Lager oder eine Welle, zur Erzeugung von Luftschall abgibt.

Die Anzeigevorrichtung zu einer für einen Anwender wahrnehmbaren Verformung der Sensorfläche ist ausgebildet, um damit die Generierung eines Stellsignals aufgrund einer Berührung der Sensorfläche oder Annäherung an die Sensorfläche anzuzeigen. Die Verformung umfasst insbesondere ein Biegen oder Wölben der Sensorfläche. Damit ist das Anzeigesignal in vorteilhafter Weise für den Anwender vorwiegend haptisch wahrnehmbar und somit insbesondere in einer Umgebung mit vielen akustischen Signalen gut unterscheidbar.

Die Sensorfläche ist insbesondere als verformbare oder biegbare Sensorfläche ausgebildet, zum Beispiel als Kunststofffläche, die insbesondere aus Polymethylmethacrylat hergestellt ist.

Vorzugsweise umfasst die Anzeigevorrichtung zumindest ein Piezoelement, das an der Sensorfläche angeordnet oder mit dieser operativ verbunden ist. Das zumindest eine Piezoelement ist ausgebildet, bei Anlegen einer elektrischen Spannung eine mechanische Bewegung auszuführen, die auf die Sensorfläche übertragen wird und diese verformt oder biegt. Nach Beendigung des Anlegens der elektrischen Spannung kehren das zumindest eine Piezoelement und die Sensorfläche in ihre Ausgangsposition zurück.

Vorzugsweise ist die Anzeigevorrichtung ausgebildet, für einen Anwender sichtbares Licht zu emittieren, insbesondere um damit die Generierung eines Stellsignals aufgrund einer Berührung der Sensorfläche oder Annäherung an die Sensorfläche anzuzeigen. Damit ist in vorteilhafter Weise eine Anzeigevorrichtung geschaffen, deren Anzeigesignal unabhängig von einer Berührung des Behandlungsinstrument, insbesondere der Sensorfläche, wahrnehmbar ist.

Vorzugsweise umfasst die Anzeigevorrichtung, welche ein für einen Anwender sichtbares Licht emittiert, zumindest eines der folgenden Elemente: eine Lichtquelle, insbesondere eine Glühbirne oder ein oder mehrere optische Halbleiterelemente, zum Beispiel eine LED; einen optischen Lichtleiter, zum Beispiel einen Glasstab oder einen Glasfaserstab; eine optisch transparente Lichtabgabefläche. Vorzugsweise sind die Lichtquelle und der optische Lichtleiter und/ oder die optisch transparente Lichtabgabefläche lichtleitend miteinander verbunden, so dass das von der Lichtquelle emittierte sichtbare Licht zur und in die optisch transparente Lichtabgabefläche leitbar ist.

Vorzugsweise bildet die optisch transparente Lichtabgabefläche zumindest einen Teil der Sensorfläche oder die gesamte Sensorfläche. Alternativ oder zusätzlich umgibt die optisch transparente Lichtabgabefläche die Sensorfläche, insbesondere ringförmig. Vorzugsweise ist die optisch transparente Lichtabgabefläche aus Glas oder Kunststoff, zum Beispiel Polymethylmethacrylat, hergestellt. Damit muss der Anwender seine Wahrnehmung in vorteilhafter Weise zum Betätigen des Stellelements und zum Erfassen des Anzeigesignals der Anzeigevorrichtung nur auf eine einzige Stelle konzentrieren.

Vorzugsweise tritt das von der Anzeigevorrichtung emittierte sichtbare, und insbesondere farbige, Licht durch die (optisch transparente) Sensorfläche oder durch die von der Sensorfläche getrennte oder beabstandete Lichtabgabefläche hindurch, um dem Anwender die Generierung eines Stellsignals anzuzeigen.

Vorzugsweise ist im Inneren des Behandlungsinstruments eine Platine angeordnet, auf der zumindest ein Teil der Anzeigevorrichtung angeordnet ist. Alternativ ist die Außenhülse aus Kunststoff gefertigt und ihre Innenseite bildet mit einer darauf angeordnete metallischen Beschichtung, insbesondere einer Kupferbeschichtung, eine Platine.

Vorzugsweise ist auf der Platine zum Beispiel der im Vorstehenden beschriebene Schwingungserzeuger oder die im Vorstehenden beschriebene zumindest eine Lichtquelle angeordnet. Vorzugsweise ist die Platine als flexible, biegbare Platine ausgebildet. Vorzugsweise ist auf der Platine auch zumindest ein Teil des Stellelements, insbesondere zumindest ein Teil des kapazitiven Sensors angeordnet. Damit ist in vorteilhafter Weise ein kompakter Aufbau des Behandlungsinstruments geschaffen.

Vorzugsweise umfasst das medizinische oder dentale, mit einer Hand haltbare Behandlungsinstrument eine, insbesondere auf der Platine angeordnete, Steuerelektronik, die mit dem kapazitiven Sensor und der Anzeigevorrichtung verbunden ist und ausgebildet ist, das Stellsignal des kapazitiven Sensors zu empfangen, ggf. zu verarbeiten und die Anzeigevorrichtung auf Basis des empfangenen Stellsignals zu steuern, um eine Berührung der Sensorfläche oder eine Annäherung an die Sensorfläche optisch, akustisch und/ oder haptisch anzuzeigen. Damit ist in vorteilhafter Weise ein Behandlungsinstrument geschaffen, das kompakt einen elektrischen Schaltkreis, der den kapazitiven Sensor, die Sensorfläche, die Anzeigevorrichtung und die Steuerelektronik umfasst, aufweist. Insbesondere ist die Steuerelektronik ausgebildet, basierend auf dem empfangenen Stellsignal ein Steuersignal zu erzeugen, um die Anzeigevorrichtung zu steuern. Insbesondere ist die Anzeigevorrichtung ausgebildet, das Steuersignal zu empfangen und auf Basis des empfangenen Steuersignals ein optisches, akustisches und/ oder haptisches Anzeigesignal zu erzeugen und abzugeben.

Vorzugsweise ist die Steuerelektronik des medizinischen oder dentalen, mit einer Hand haltbaren Behandlungsinstruments mit einem Stellglied verbunden und dafür vorgesehen, auf Basis des empfangenen Stellsignals des kapazitiven Sensors das Stellglied, insbesondere eines der im Vorstehenden genannten Stellglieder, zu steuern. Vorzugsweise umfasst die Steuerelektronik einen Controller, um zum Beispiel die Datenübertragung zu vereinfachen. Vorzugsweise ist das Stellglied Teil des Behandlungsinstruments und/ oder an diesem angeordnet. Vorzugsweise umfasst das Steuern das Ein- und Ausschalten des Stellglieds und/ oder das Stellen eines Stellwerts eines Stellglieds stufenlos oder in Stufen, so wie dies im Vorstehenden beschrieben ist. Damit ist in vorteilhafter Weise ein Behandlungsinstrument mit einem eigenständigen Schaltkreis für die Anzeigevorrichtung und das Stellglied geschaffen.

Vorzugsweise umfasst das medizinische oder dentale, mit einer Hand haltbare Behandlungsinstrument eine Übertragungsvorrichtung, die ausgebildet ist, das Stellsignal oder ein durch das Stellsignal getriggertes Steuersignal an einen externen (i.e. außerhalb des mit einer Hand haltbaren Behandlungsinstruments) Controller zu übertragen. Damit ist es in vorteilhafter Weise möglich, das Stellsignal und/ oder das Steuersignal außerhalb des mit einer Hand haltbaren Behandlungsinstrument zu verarbeiten, speichern und/ oder anzuzeigen. Vorzugsweise ist der externe Controller in einer Dentaleinheit oder in einem Tisch-Steuergerät vorgesehen.

Insbesondere ist der externe Controller ausgebildet, das Stellsignal des kapazitiven Sensors zu empfangen, ggf. zu verarbeiten und die Anzeigevorrichtung des Behandlungsinstruments auf Basis des empfangenen Stellsignals zu steuern, um eine Berührung der Sensorfläche oder eine Annäherung an die Sensorfläche optisch, akustisch und/ oder haptisch anzuzeigen. Alternativ oder zusätzlich ist der externe Controller mit einem Stellglied verbunden und dafür vorgesehen, auf Basis des empfangenen Stellsignals des kapazitiven Sensors oder eines dadurch getriggerten Steuersignals das Stellglied, insbesondere eines der im Vorstehenden genannten und an dem Behandlungsinstrument vorgesehenen Stellglieder, zu steuern. Alternativ oder zusätzlich ist der externe Controller ausgebildet, auf Basis des empfangenen Stellsignals des kapazitiven Sensors oder eines dadurch getriggerten Steuersignals eine an dem externen Controller oder der Dentaleinheit oder dem Tisch-Steuergerät vorgesehene externe Anzeigevorrichtung zu steuern, um zum Beispiel das Stellen des Stellglied anzuzeigen.

Vorzugsweise umfasst die Übertragungsvorrichtung zumindest eines der folgenden Elemente: eine, insbesondere mechanische, Kupplungsvorrichtung; einen Schlauch; ein elektrische oder optische Leitung; ein Kabel; einen drahtlosen Transmitter; eine Funkverbindung.

Vorzugsweise sind die im Vorstehenden beschriebenen optischen, akustischen und/ oder haptischen Anzeigesignale durch die Steuerelektronik oder den Controller zeitlich begrenzt, insbesondere auf einen Zeitraum von unter einer Sekunde oder wenige Sekunden, zum Beispiel in einem Bereich von 0,1 sec - 5 sec.

Die Erfindung wird nachfolgend anhand bevorzugter Ausführungsbeispiele und Bezug nehmend auf die beigefügten Zeichnungen erläutert:

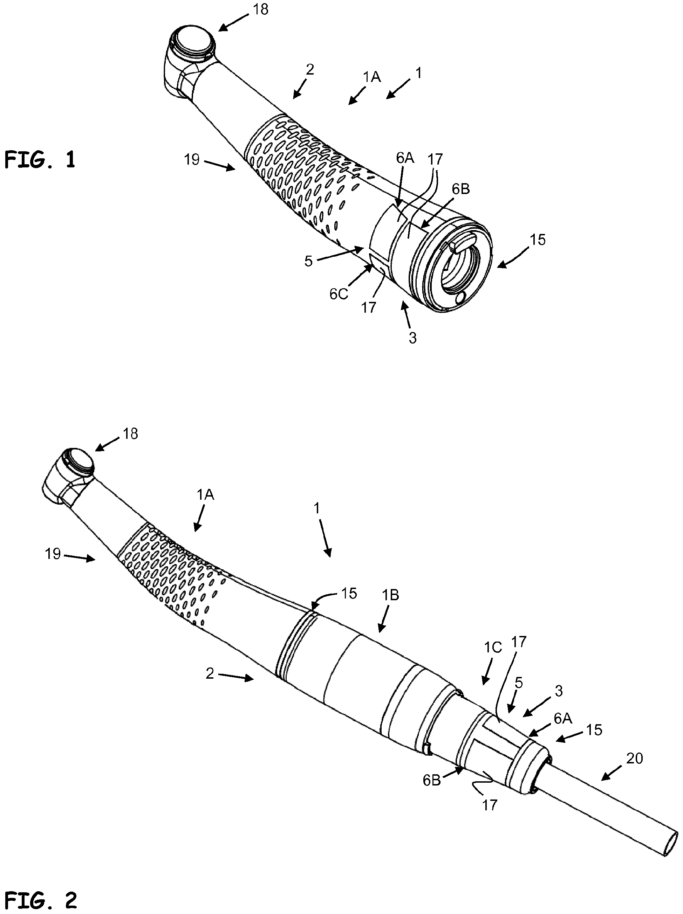

Figur 1 zeigt ein erstes Beispiel eines medizinischen oder dentalen, mit einer Hand haltbaren Behandlungsinstruments mit einem Stellelement mit einem kapazitiven Sensor und einer Anzeigevorrichtung, welche die Sensorflächen des kapazitiven Sensors und die Generierung eines Stellsignals anzeigt;
Figur 2 zeigt ein zweites Beispiel, das nicht von der Erfindung abgedeckt wird, eines medizinischen oder dentalen, mit einer Hand haltbaren Behandlungsinstruments mit einem Stellelement mit einem kapazitiven Sensor und einer Anzeigevorrichtung in Form von Markierungen, welche die Sensorflächen des kapazitiven Sensors anzeigen;

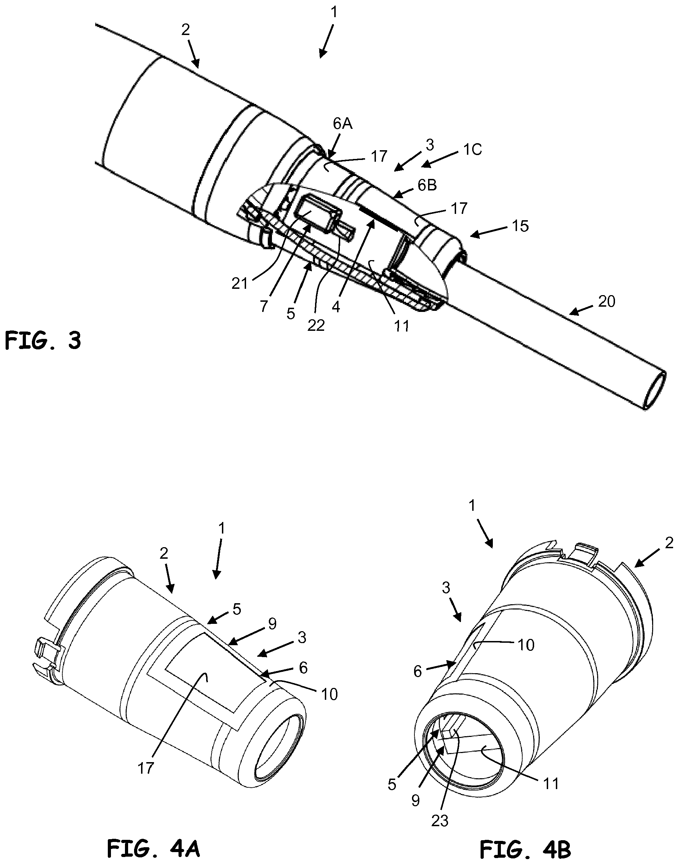

Figur 3 zeigt ein Ausführungsbeispiel eines medizinischen oder dentalen, mit einer Hand haltbaren Behandlungsinstruments mit einem Stellelement mit einem kapazitiven Sensor und einer Anzeigevorrichtung mit einem Schwingungserzeuger, welche die Sensorflächen des kapazitiven Sensors und die Generierung eines Stellsignals anzeigt;
Figuren 4A und 4B zeigen ein weiteres Beispiel, das nicht von der Erfindung abgedeckt ist, eines medizinischen oder dentalen, mit einer Hand haltbaren Behandlungsinstruments mit einem Stellelement mit einem kapazitiven Sensor und einer optischen Anzeigevorrichtung, welche die Sensorflächen des kapazitiven Sensors und die Generierung eines Stellsignals anzeigt;

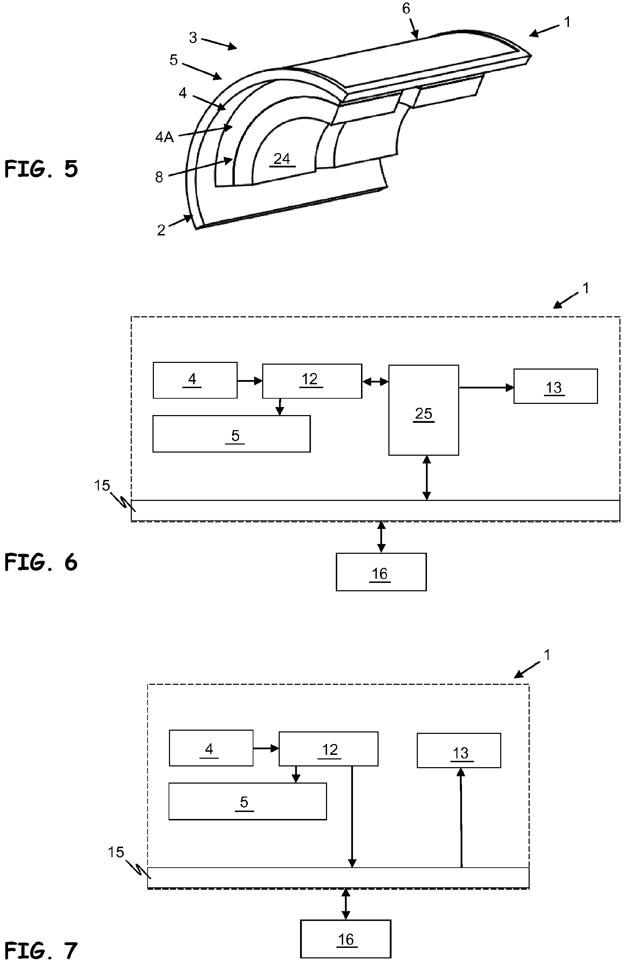

Figur 5 zeigt ein weiteres Beispiel, das nicht von der Erfindung abgedeckt ist, eines medizinischen oder dentalen, mit einer Hand haltbaren Behandlungsinstruments mit einem Stellelement mit einem kapazitiven Sensor und einer Anzeigevorrichtung, welche die Sensorflächen des kapazitiven Sensors und die Generierung eines Stellsignals durch Verformung der Sensorfläche anzeigt;
Figur 6 zeigt in einem Blockbild ein Ausführungsbeispiel einer Signalübertragung und Signalverarbeitung eines medizinischen oder dentalen, mit einer Hand haltbaren Behandlungsinstruments mit einem Stellelement mit einem kapazitiven Sensor und einer Anzeigevorrichtung, welche die Generierung eines Stellsignals anzeigt;
Figur 7 zeigt in einem Blockbild ein alternatives Ausführungsbeispiel einer Signalübertragung und Signalverarbeitung eines medizinischen oder dentalen, mit einer Hand haltbaren Behandlungsinstruments mit einem Stellelement mit einem kapazitiven Sensor und einer Anzeigevorrichtung, welche die Generierung eines Stellsignals anzeigt.

Das in der Figur 1 dargestellte medizinische oder dentale, mit einer Hand haltbare Behandlungsinstrument 1 ist als Winkelstück 1A ausgebildet. Es umfasst einen Kopfteil 18 und einen daran anschließenden, gebogenen Griffteil 19. Im Kopfteil 18 ist zumindest eine Werkzeughalterung beweglich angeordnet, um ein medizinisches oder dentales Behandlungswerkzeug lösbar aufzunehmen. Im Kopfteil 18 und/ oder Griffteil 19 ist des Weiteren eine Antriebsvorrichtung vorgesehen, die zum Beispiel eine oder mehrere Wellen, ein Getriebe, eine Druckgasleitung, ein durch Druckgas antreibbares Laufrad oder ähnliches aufweist. Die Antriebsvorrichtung ist operativ mit der Werkzeughalterung verbunden, um diese und das darin gehaltene Werkzeug in Bewegung zu setzen.

Das Behandlungsinstrument 1 weist eine mehrteilige Außenhülse 2 auf, die einen am Kopfteil 18 vorgesehenen Kopfhülsenteil und einen am Griffteil 19 vorgesehenen, gebogenen Griffhülsenteil aufweist.

Am dem Kopfteil 18 abgewandten Ende des Behandlungsinstrument 1 ist eine Kupplungsvorrichtung oder Übertragungsvorrichtung 15 vorgesehen, die mit einer Versorgungseinheit oder Dentaleinheit oder einem externen Controller 16 (siehe Figuren 6, 7) lösbar verbindbar ist. Über die Kupplungsvorrichtung 15 ist/ sind zumindest ein Medium, zum Beispiel elektrische Energie, Druckgas, insbesondere Druckluft, Wasser, Licht und/ oder Daten, zum Beispiel Messdaten, Steuer- oder Regeldaten, Identifikationsdaten, zum Beispiel zum Identifizieren des Behandlungsinstruments 1 oder des damit verbundenen Werkzeugs, Betriebsdaten oder ähnliche Daten, übertragbar. Die Übertragung des zumindest einen Mediums und/ oder der Daten zwischen dem Behandlungsinstrument 1 und der Versorgungseinheit oder Dentaleinheit oder dem externen Controller 16 kann unidirektional oder bidirektional ausgebildet sein.

An dem Griffteil 19, insbesondere am kupplungsseitige Ende des Griffteils 19, oder an der Kupplungsvorrichtung 15 ist an der Außenhülse 2 ein Stellelement 3 vorgesehen. Das Stellelement 3 umfasst mehrere kapazitive Sensoren 4 (siehe Figur 3) und drei an der Außenhülse 2 vorgesehene und operativ mit dem kapazitiven Sensor 4 verbundene Sensorflächen 6A, 6B, 6C, bei deren Berührung oder bei einer Annäherung daran das Stellelement 3 Stellsignale generiert, um damit ein oder mehrere Stellglieder 13 (siehe Figuren 6, 7) zu steuern.

Bespielhaft ist die Sensorfläche 6A dafür vorgesehen, ein erstes Stellglied 13, zum Beispiel einen Motor zum Antreiben eines mit dem Behandlungsinstrument 1 verbindbaren Werkzeugs, ein- und auszuschalten. Beispielhaft ist die Sensorfläche 6B dafür vorgesehen, einen Parameter des ersten Stellglieds 13, zum Beispiel die Drehzahl oder Leistung des Motors, stufenlos oder in vorgegebenen Schritten zu stellen. Beispielhaft ist die Sensorfläche 6C dafür vorgesehen ein zweites Stellglied, zum Beispiel eine Beleuchtungsvorrichtung des Behandlungsinstrument 1 zur Abgabe elektromagnetischer Strahlung in Richtung der Behandlungsstelle, ein- und auszuschalten.

Die Sensorflächen 6A, 6B, 6C sind als Teil oder Abschnitt der Außenhülse 2, insbesondere einstückig mit der Außenhülse 2, ausgebildet. Somit sind die Sensorflächen 6A, 6B, 6C und/ oder die Grenzen der Sensorflächen 6A, 6B, 6C für einen Anwender bei einem Blick auf die Außenhülse 2 schwer oder nicht erkennbar und eine zuverlässige Bedienbarkeit des Stellelements 3 ist nicht gewährleistet.

Um die Sensorflächen 6A, 6B, 6C für einen Anwender erkennbar zu machen, ist daher an dem Behandlungsinstrument 1 eine Anzeigevorrichtung 5 vorgesehen, welche die Sensorfläche 6 und/ oder deren Außengrenzen anzeigt. Die Anzeigevorrichtung 5 umfasst drei Markierungen 17, zum Beispiel Farbmarkierungen, die um, auf oder über den Sensorflächen 6A, 6B, 6C auf der Außenhülse 2 angeordnet sind, wobei jeweils eine Markierung 17 einer der drei Sensorflächen 6A, 6B, 6C zugeordnet ist. Damit wird eine zuverlässige Bedienbarkeit des Stellelements 3 und der drei Sensorflächen 6A, 6B, 6C ermöglicht.

Die Anzeigevorrichtung 5 des Behandlungsinstruments 1 ist des Weiteren dafür vorgesehen, die Generierung eines Stellsignals aufgrund einer Berührung der Sensorflächen 6A, 6B, 6C oder einer Annäherung an diese anzuzeigen. Somit erhält der Anwender eine Rückmeldung, ob das Stellelement 3 ein Stellsignal generiert hat oder nicht. Die Anzeigevorrichtung 5 erzeugt dazu ein haptisches Anzeigesignal und gibt dieses ab, insbesondere an die Sensorflächen 6A, 6B, 6C, besonders bevorzugt nur an die vom Anwender beim Stellvorgang verwendete Sensorfläche 6A, 6B, 6C. Die Anzeigevorrichtung 5 kann insbesondere so aufgebaut sein, wie es im Folgenden in Bezug auf die Figuren 3 - 5 detaillierter beschrieben ist und die dort genannten Bauteil aufweisen.

Das in der Figur 2 dargestellte medizinische oder dentale, mit einer Hand haltbare Behandlungsinstrument 1 umfasst ein Winkelstück 1A, einen motorischen Antrieb 1B und eine Kupplungseinheit 1C.

Das Winkelstück 1A weist ein Kopfteil 18, ein Griffteil 19 und eine Kupplungsvorrichtung oder Übertragungsvorrichtung 15 auf und gleicht in Bezug auf diese Bauteile dem Winkelstück 1A der Figur 1, auf deren Beschreibung verwiesen wird. Das Winkelstück 1A der Figur 2 weist jedoch kein Stellelement 3 und keine Anzeigevorrichtung 5 auf.

Der motorische Antrieb 1B ist als separates Bauteil ausgebildet, das über ein oder mehrere (winkelstückseitige) Kupplungselemente mit der Kupplungsvorrichtung 15 des Winkelstücks 1A lösbar verbindbar ist. Alternativ ist es auch möglich, den motorischen Antrieb 1B als integralen, für den Anwender nicht entfernbaren Teil des Winkelstücks 1A auszubilden. Der motorische Antrieb 1B umfasst insbesondere einen Elektromotor, der mit im Winkelstück 1A angeordneten Wellen und/ oder Getrieben verbindbar ist, um diese und ein damit verbindbares Behandlungswerkzeug in eine Antriebsbewegung zu versetzen.

An seinem dem Winkelstück 1A abgewandten Ende ist der motorische Antrieb 1B mit der Kupplungseinheit oder Schlauchkupplung 1C verbunden. Die Kupplungseinheit 1C ist als Übertragungsvorrichtung 15 ausgebildet, welche das Winkelstück 1A und den motorischen Antrieb 1B über einen Schlauch 20 mit einer Steuer- und/ oder Regel- und/ oder Versorgungseinheit oder Dentaleinheit oder einem externen Controller 16 zwecks Austausch von Daten, Energie und/ oder Medien zu verbinden. In dem Schlauch 20 ist/ sind ein oder mehrere elektrische und/ oder optische Leitungen und/ oder Medienleitungen zur Übertragung der Daten, Energie und/ oder Medien angeordnet.

An der Außenhülse 2 der Kupplungseinheit 1C ist des Weiteren ein Stellelement 3 mit zumindest einem kapazitiven Sensor 4 und zwei an der Außenhülse 2 vorgesehenen und operativ mit dem zumindest einen kapazitiven Sensor 4 verbundenen Sensorflächen 6A, 6B angeordnet. Bei Berührung oder bei einer Annäherung an die Sensorflächen 6A, 6B mit einem Bedienelement, zum Beispiel einem Finger eines Anwenders, generiert das Stellelement 3 Stellsignale, um damit ein oder mehrere Stellglieder 13 zu steuern.

Eine Anzeigevorrichtung 5 umfasst zwei Markierungen 17, zum Beispiel Farbmarkierungen oder haptisch wahrnehmbare Markierungen, die um, auf oder über den Sensorflächen 6A, 6B angeordnet oder als Teil der Sensorflächen 6A, 6B ausgebildet sind. Jeweils eine Markierung 17 ist einer der beiden Sensorflächen 6A, 6B zugeordnet. Damit wird eine zuverlässige Bedienbarkeit des Stellelements 3 und der Sensorflächen 6A, 6B ermöglicht.

Das in der Figur 3 dargestellte medizinische oder dentale, mit einer Hand haltbare Behandlungsinstrument 1 umfasst wiederum eine Kupplungseinheit 1C, die insbesondere als Übertragungsvorrichtung 15 ausgebildet ist, um Daten, Energie und/ oder Medien von einer Steuer- und/ oder Regel- und/ oder Versorgungseinheit oder Dentaleinheit oder einem externen Controller 16 zu übertragen und/ oder an diese(n) zu leiten.

Die Kupplungseinheit 1C weist ein Stellelement 3 mit zumindest einem kapazitiven Sensor 4 und mehreren an der Außenhülse 2 vorgesehenen und operativ mit dem zumindest einen kapazitiven Sensor 4 verbundenen Sensorflächen 6A, 6B auf. Eine Anzeigevorrichtung 5 umfasst Markierungen 17, zum Beispiel Farbmarkierungen oder haptisch wahrnehmbare Markierungen, die um, auf oder über den Sensorflächen 6A, 6B angeordnet oder als Teil der Sensorflächen 6A, 6B ausgebildet sind, um die Sensorflächen 6A, 6B und/ oder deren Außengrenzen anzuzeigen. Jeweils eine Markierung 17 ist einer der Sensorflächen 6A, 6B zugeordnet.

Zusätzlich ist die Anzeigevorrichtung 5 dafür vorgesehen, die Generierung eines Stellsignals aufgrund einer Berührung der Sensorflächen 6A, 6B oder einer Annäherung an diese anzuzeigen. Die Anzeigevorrichtung 5 erzeugt dafür ein haptisches Anzeigesignal und gibt dieses ab, insbesondere an die Sensorflächen 6A, 6B, besonders bevorzugt nur an die vom Anwender beim Stellvorgang verwendete Sensorfläche 6A, 6B.

Die haptische Anzeigevorrichtung 5 umfasst einen Schwingungserreger 7. Der Schwingungserreger 7 weist einen Unwuchtmotor mit einem Elektromotor 21 und einer exzentrisch zu einer Rotationsachse einer Rotorwelle des Elektromotors 21 angeordneten Masse 22 auf.

Der Schwingungserreger 7 ist auf einer Platine 11 im Inneren der Kupplungseinheit 1C befestigt. Die Platine 11 ist direkt oder indirekt mit der Innenseite der Außenhülse 2 der Kupplungseinheit 1C verbunden, so dass die von dem Schwingungserreger 7 erzeugten Schwingungen über die Platine 11 an die Außenhülse 2, insbesondere an die Sensorflächen 6A, 6B, übertragbar und für den Anwender wahrnehmbar sind. Der Anwender erhält somit eine haptische Rückmeldung über die Betätigung des Stellelements 3 und/ oder die Generierung eines Stellsignals.

Die Figuren 4A, 4B zeigen eine Außenhülse 2 eines medizinischen oder dentalen, mit einer Hand haltbaren Behandlungsinstruments 1. An der Außenhülse 2 ist ein Stellelement 3 mit zumindest einem kapazitiven Sensor 4 und einer an der Außenhülse 2 vorgesehenen und operativ mit dem zumindest einen kapazitiven Sensor 4 verbundenen Sensorflächen 6 vorgesehen.

Eine Anzeigevorrichtung 5 umfasst eine Markierungen 17, die auf der Sensorfläche 6 angeordnet oder als Teil der Sensorfläche 6 ausgebildet ist, um die Sensorflächen 6 und/ oder deren Außengrenzen anzuzeigen. Die Anzeigevorrichtung 5 ist auch dafür vorgesehen, die Generierung eines Stellsignals aufgrund einer Berührung der Sensorfläche 6 oder einer Annäherung an diese anzuzeigen. Die Anzeigevorrichtung 5 ist dafür als optische Anzeigevorrichtung 9 ausgebildet und erzeugt ein optisches Anzeigesignal, insbesondere für den Anwender sichtbares Licht. Das sichtbare Licht tritt durch eine von der Sensorfläche 6 getrennte und an die Sensorfläche 6 angrenzend Lichtabgabefläche 10 aus der Außenhülse 2 nach außen.

Die Lichtabgabefläche 10 ist aus Glas oder Kunststoff gebildet und umgibt die Sensorfläche 6 und/ oder den kapazitiven Sensor 4 rahmenförmig. Die Lichtabgabefläche 10 und die Sensorfläche 6 sind in eine Öffnung der Außenhülse 2, insbesondere bündig, aufgenommen.

Die optische Anzeigevorrichtung 9 umfasst eine Lichtquelle 23, insbesondere zumindest ein optisches Halbleiterelement, zum Beispiel eine LED, die auf einer Platine 11 befestigt ist und über die Platine 11 mit elektrischer Energie versorgbar ist. Zumindest eine Teil des sichtbaren Lichts wird von der Lichtquelle 23 in Richtung der Lichtabgabefläche 10 emittiert, um durch die Lichtabgabefläche 10 nach außen geleitet zu werden.

Die Figur 5 zeigt eine Außenhülse 2 eines medizinischen oder dentalen, mit einer Hand haltbaren Behandlungsinstruments 1. An der Außenhülse 2 ist ein Stellelement 3 mit zumindest einem kapazitiven Sensor 4 und einer an der Außenhülse 2 vorgesehenen und operativ mit dem zumindest einen kapazitiven Sensor 4 verbundenen Sensorflächen 6 vorgesehen.

Der kapazitive Sensor 4 umfasst eine Metallschicht 4A, zum Beispiel eine Kupferschicht, so wie dies zum Beispiel auch für die kapazitiven Sensoren 4 der Figuren 1-4B zutreffen kann. Die Metallschicht 4A ist an der Innenseite der Sensorfläche 6 angeordnet, zum Beispiel aufgedampft.

Eine Anzeigevorrichtung 5, 8 ist ausgebildet, die Generierung eines Stellsignals aufgrund einer Berührung der Sensorfläche 6 oder einer Annäherung an diese durch eine für einen Anwender wahrnehmbare Verformung der Sensorfläche 6 anzuzeigen. Dazu ist zumindest ein Piezoelement 24 vorgesehen, das an der Metallschicht 4A angeordnet und dadurch mit der Sensorfläche 6 operativ verbunden ist. Das zumindest eine Piezoelement 24 ist ausgebildet, bei Anlegen einer elektrischen Spannung eine mechanische Bewegung auszuführen, die auf die Sensorfläche 6 übertragen wird und diese verformt oder biegt.

Die Figuren 6 und 7 zeigen schematisch zwei unterschiedliche Ausführungsbeispiele einer Signalübertragung und Signalverarbeitung medizinischer oder dentaler, mit einer Hand haltbarer Behandlungsinstrumente 1 mit jeweils einem Stellelement 3 mit einem kapazitiven Sensor 4 und einer Anzeigevorrichtung 5, welche die die Generierung eines Stellsignals anzeigt. Die Anzeigevorrichtung 5 ist vorzugsweise durch eine der in den Figuren 1 - 5 dargestellten und in der zugehörigen Beschreibung beschriebenen Anzeigevorrichtungen 5 gebildet.

Das Bezugszeichen 12 repräsentiert eine im Behandlungsinstrument 1 angeordnete Steuerelektronik, die mit dem kapazitiven Sensor 4 und der Anzeigevorrichtung 5 verbunden ist und ausgebildet ist, das Stellsignal des kapazitiven Sensors 4 zu empfangen und die Anzeigevorrichtung 5 auf Basis des empfangenen Stellsignals zu steuern, um eine Berührung der Sensorfläche 6 - 6C oder eine Annäherung daran anzuzeigen. Die Aktivierung der Anzeigevorrichtung 5, zum Beispiel die Abgabe von sichtbarem Licht oder von Schwingungen oder das Ausführen einer Bewegung, um damit insbesondere ein Verformen der Sensorfläche 6 zu bewirken, ist somit in beiden Ausführungsbeispielen der Figuren 6 und 7 durch die im Behandlungsinstrument 1, insbesondere auf der Platine 11, angeordnete Steuerelektronik 12 bewirkt.

Gemäß der Figur 7 ist das Behandlungsinstrument 1, insbesondere die Kupplungsvorrichtung oder Übertragungsvorrichtung 15, ausgebildet, das Stellsignal des kapazitiven Sensors 4 an eine Versorgungseinheit oder Dentaleinheit oder einen externen Controller 16 zu übertragen. Die Einheit oder der externe Controller 16 ist ausgebildet, das Stellsignal des kapazitiven Sensors 4 oder ein durch das Stellsignal getriggertes Steuersignal zu empfangen, ggf. zu verarbeiten und ein Stellglied 13 auf Basis des empfangenen Stellsignals zu steuern. Dazu generiert die Einheit oder der Controller 16 ein Stellglied-Steuersignal, das über die Kupplungsvorrichtung oder Übertragungsvorrichtung 15 an das Stellglied 13 übertragbar ist.

Gemäß dem alternativen Ausführungsbeispiel der Figur 6 ist im Behandlungsinstrument 1 ein interner Controller 25 vorgesehen, der ausgebildet ist, das Stellsignal des kapazitiven Sensors 4 oder ein durch das Stellsignal getriggertes Steuersignal zu empfangen, ggf. zu verarbeiten und ein Stellglied 13 auf Basis des empfangenen Stellsignals zu steuern. Dazu generiert der interne Controller 25 ein Stellglied-Steuersignal, das an das Stellglied 13 übertragbar ist.

Optional ist das Stellsignal des kapazitiven Sensors 4 oder das Stellglied-Steuersignal des internen Controllers 25 über die Kupplungsvorrichtung oder Übertragungsvorrichtung 15 an eine Versorgungseinheit oder Dentaleinheit oder einen externen Controller 16 übertragbar. Die Einheit oder der externe Controller 16 ist ausgebildet, das Stellsignal oder das Stellglied-Steuersignal zu empfangen, ggf. zu verarbeiten und auf Basis des empfangenen Signals eine externe Vorrichtung zu steuern. Die externe Vorrichtung umfasst zum Beispiel eine externe Anzeigevorrichtung, auf der die Betätigung des Stellglieds 13 oder ein Wert eines Betriebsparameters des Stellglieds 13 dargestellt wird.

## Patentansprüche

1. Medizinisches oder dentales, mit einer Hand haltbares Behandlungsinstrument (1), umfassend: eine ein- oder mehrteilige Außenhülse (2) und zumindest ein an der Außenhülse (2) vorgesehenes Stellelement (3) mit zumindest einem kapazitiven Sensor (4) und einer an der Außenhülse (2) vorgesehenen und operativ mit dem kapazitiven Sensor (4) verbundenen Sensorfläche (6 - 6C), bei deren Berührung oder bei einer Annäherung daran das Stellelement (3) ein Stellsignal generiert, wobei eine Anzeigevorrichtung (5) mit einem Schwingungserzeuger (7), der Schwingungen in Form von Körperschall erzeugt vorgesehen ist, wobei die Anzeigevorrichtung (5) an dem mit einer Hand haltbaren Behandlungsinstrument (1) angeordnet ist und die Generierung eines Stellsignals aufgrund einer Berührung der Sensorfläche (6 - 6C) oder Annäherung an die Sensorfläche (6 - 6C) durch die Erzeugung der Körperschallschwingungen und Übertragung der Körperschallschwingungen auf eine Anzeigefläche der Anzeigevorrichtung (5) und/ oder die Sensorfläche (6 - 6C) anzeigt.

2. Medizinisches oder dentales, mit einer Hand haltbares Behandlungsinstrument (1) nach Anspruch 1, **dadurch gekennzeichnet, dass**
die Anzeigefläche der Anzeigevorrichtung (5) von der Sensorfläche (6 - 6C) getrennt ist und diese umgibt.

3. Medizinisches oder dentales, mit einer Hand haltbares Behandlungsinstrument (1) nach Anspruch 1 oder 2, **dadurch gekennzeichnet, dass**
der Schwingungserzeuger (7) der Anzeigevorrichtung (5) ein Piezoelement aufweist.

4. Medizinisches oder dentales, mit einer Hand haltbares Behandlungsinstrument (1) nach einem der vorstehenden Ansprüche, **dadurch gekennzeichnet, dass** der Schwingungserzeuger (7) der Anzeigevorrichtung (5) einen Unwuchtmotor aufweist.

5. Medizinisches oder dentales, mit einer Hand haltbares Behandlungsinstrument (1) nach einem der vorstehenden Ansprüche, **dadurch gekennzeichnet, dass**
die Anzeigevorrichtung (5) zur Erzeugung von Schwingungen über ein Bauteil der Anzeigevorrichtung (5) und/ oder über zumindest ein Bauteil des Behandlungsinstruments (1) operativ mit der Anzeigefläche oder der Sensorfläche (6 - 6C) verbunden ist, um Schwingungen auf die Anzeigefläche oder die Sensorfläche (6 - 6C) zu übertragen.

6. Medizinisches oder dentales, mit einer Hand haltbares Behandlungsinstrument (1) nach einem der vorstehenden Ansprüche, **dadurch gekennzeichnet, dass**
die Anzeigevorrichtung (5) zur Erzeugung von Schwingungen für einen Anwender akustisch wahrnehmbare Schwingungen erzeugt.

7. Medizinisches oder dentales, mit einer Hand haltbares Behandlungsinstrument (1) nach einem der vorstehenden Ansprüche, **gekennzeichnet durch**
eine im Inneren des Behandlungsinstruments (1) angeordnete Platine (11), auf der zumindest ein Teil der Anzeigevorrichtung (5) angeordnet ist.

8. Medizinisches oder dentales, mit einer Hand haltbares Behandlungsinstrument (1) nach Anspruch 7, **dadurch gekennzeichnet, dass**
auf der Platine (11) zusätzlich zumindest ein Teil des Stellelements (3), insbesondere zumindest ein Teil des kapazitiven Sensors (4), angeordnet ist.

9. Medizinisches oder dentales, mit einer Hand haltbares Behandlungsinstrument (1) nach einem der vorstehenden Ansprüche, **gekennzeichnet durch**
eine im Behandlungsinstrument (1) angeordnete Steuerelektronik (12), die mit dem kapazitiven Sensor (4) und der Anzeigevorrichtung (5) verbunden ist und ausgebildet ist, das Stellsignal des kapazitiven Sensors (4) zu empfangen und die Anzeigevorrichtung (5) auf Basis des empfangenen Stellsignals zu steuern, um eine Berührung der Sensorfläche (6 - 6C) oder eine Annäherung an die Sensorfläche (6 - 6C) anzuzeigen.

10. Medizinisches oder dentales, mit einer Hand haltbares Behandlungsinstrument (1) nach Anspruch 9, **dadurch gekennzeichnet, dass**
die Steuerelektronik (12) des Weiteren mit einem Stellglied (13) verbunden ist und dafür vorgesehen ist, auf Basis des empfangenen Stellsignals des kapazitiven Sensors (4) das Stellglied (13) zu steuern.

11. Medizinisches oder dentales, mit einer Hand haltbares Behandlungsinstrument (1) nach einem der vorstehenden Ansprüche, **gekennzeichnet durch**
eine Übertragungsvorrichtung (15), die ausgebildet ist, das Stellsignal oder ein durch das Stellsignal getriggertes Steuersignal an einen externen Controller (16) zu übertragen.

12. Medizinisches oder dentales, mit einer Hand haltbares Behandlungsinstrument (1) nach einem der vorstehenden Ansprüche, **dadurch gekennzeichnet, dass**
die Anzeigevorrichtung (5) eine an der Oberfläche der Außenhülse (2) für den Anwender sichtbare und/ oder fühlbare Markierung (17) aufweist.

13. Medizinisches oder dentales, mit einer Hand haltbares Behandlungsinstrument (1) nach Anspruch 12, **dadurch gekennzeichnet, dass**
die Markierung (17) zumindest eines der folgenden Elemente aufweist: eine Farbmarkierung; eine für einen Anwender haptisch wahrnehmbare Markierung, die zur haptischen Wahrnehmung zumindest eine Vertiefung und/ oder Erhebung aufweist.

14. Medizinisches oder dentales, mit einer Hand haltbares Behandlungsinstrument (1) nach Anspruch 12 oder 13, **dadurch gekennzeichnet, dass** die Markierung (17) auf der Außenhülse (2) und/ oder der Sensorfläche (6 - 6C) befestigt oder aufgetragen oder an oder durch die Außenhülse (2) und/ oder Sensorfläche (6 - 6C) gebildet ist.

15. Medizinisches oder dentales, mit einer Hand haltbares Behandlungsinstrument (1) nach einem der vorstehenden Ansprüche, **dadurch gekennzeichnet, dass**
das Stellelement (3) und die Anzeigevorrichtung (5) an einem Schlauchkupplungsabschnitt (1C) des Behandlungsinstruments (1) zur Verbindung mit einer Medienquelle, Energiequelle, Steuer- oder Regelvorrichtung (16) vorgesehen sind.

## Claims

1. Medical or dental hand-held treatment instrument (1), comprising: a single-part or multi-part outer sleeve (2) and at least one controlling element (3) provided on the outer sleeve (2) and having at least one capacitive sensor (4) and a sensor surface (6 - 6C) which is provided on the outer sleeve (2) and is operatively connected to the capacitive sensor (4), wherein the controlling element (3) generates an actuating signal when the sensor surface (6 - 6C) is touched or approached, wherein
an indicating device (5) is provided which has a vibration generator (7) generating vibrations in the form of structure-borne sound, wherein the indicating device (5) is arranged on the hand-held treatment instrument (1) and indicates the generation of an actuating signal as a result of contact with the sensor surface (6 - 6C) or approach to the sensor surface (6 - 6C) by generating the structure-borne sound vibrations and transmission of the structure-borne sound vibrations to an indication surface of the indicating device (5) and/or to the sensor surface (6 - 6C).

2. Medical or dental hand-held treatment instrument (1) according to claim 1, **characterized in that**
the indication surface of the indicating device (5) is separated from the sensor surface (6 - 6C) and surrounds the same.

3. Medical or dental hand-held treatment instrument (1) according to claim 1 or 2, **characterized in that**
the vibration generator (7) of the indicating device (5) comprises a piezo element.

4. Medical or dental hand-held treatment instrument (1) according to any one of the preceding claims, **characterized in that**
the vibration generator (7) of the indicating device (5) comprises an unbalance motor.

5. Medical or dental hand-held treatment instrument (1) according to any one of the preceding claims, **characterized in that**
the indicating device (5) for generating vibrations is operatively connected via a component of the indicating device (5) and/or at least one component of the treatment instrument (1) to the indication surface or to the sensor surface (6 - 6C) to transmit vibrations to the indication surface or to the sensor surface (6 - 6C).

6. Medical or dental hand-held treatment instrument (1) according to any one of the preceding claims, **characterized in that**
the indicating device (5) for generating vibrations generates vibrations that are acoustically perceptible to a user.

7. Medical or dental hand-held treatment instrument (1) according to any one of the preceding claims, **characterized by**
a circuit board (11) arranged inside the treatment instrument (1) and on which at least part of the indicating device (5) is arranged.

8. Medical or dental hand-held treatment instrument (1) according to claim 7, **characterized in that**
at least a part of the controlling element (3), in particular at least a part of the capacitive sensor (4) is arranged in addition on the circuit board (11).

9. Medical or dental hand-held treatment instrument (1) according to any one of the preceding claims, **characterized by**
control electronics (12) arranged in the treatment instrument (1), which are connected to the capacitive sensor (4) and the indicating device (5) and are configured to receive the actuating signal of the capacitive sensor (4) and to control the indicating device (5) on the basis of the received actuating signal in order to indicate a contact with the sensor surface (6 - 6C) or an approach to the sensor surface (6 - 6C).

10. Medical or dental hand-held treatment instrument (1) according to claim 9, **characterized in that**
the control electronics (12) are further connected to an actuator (13) and are provided for controlling the actuator (13) on the basis of the received actuating signal of the capacitive sensor (4).

11. Medical or dental hand-held treatment instrument (1) according to any one of the preceding claims, **characterized in that**
a transmission device (15) which is configured to transmit the actuating signal, or a control signal triggered by the actuating signal to an external controller (16).

12. Medical or dental hand-held treatment instrument (1) according to any one of the preceding claims, **characterized in that**
the indicating device (5) comprises a marking (17) on the surface of the outer sleeve (2) that is visible and/or tactile to the user.

13. Medical or dental hand-held treatment instrument (1) according to claim 12, **characterized in that**
the marking (17) comprises at least one of the following elements: a color marking; a marking that is haptically perceptible to a user and comprises at least one depression and/or elevation for haptic perception.

14. Medical or dental hand-held treatment instrument (1) according to claim 12 or 13, **characterized in that**
the marking (17) is fixed on or applied to the outer sleeve (2) and/or the sensor surface (6 - 6C) or is formed on or by the outer sleeve (2) and/or sensor surface (6 - 6C).

15. Medical or dental hand-held treatment instrument (1) according to any one of the preceding claims, **characterized in that**
the controlling element (3) and the indicating device (5) are provided on a hose coupling section (1C) of the treatment instrument (1) for connection to a media source, energy source, control or regulating device (16).

## Revendications

1. Instrument de traitement (1) médical ou dentaire tenant dans une main, comprenant:
une gaine extérieure (2) en une ou en plusieurs parties, au moins un élément de réglage (3) prévu sur la gaine extérieure (2), pourvu d'au moins un capteur (4) capacitif et une surface de capteur (6 - 6C) prévue sur la gaine extérieure (2) et opérationnellement connectée avec le capteur (4) capacitif, au toucher ou à l'approche de laquelle l'élément de réglage (3) génère un signal de réglage,
un dispositif d'affichage (5) avec un générateur de vibrations (7), qui génère des vibrations sous la forme de bruits de structure, est prévu, le dispositif d'affichage (5) étant placé sur l'instrument de traitement (1) tenant dans une main et affichant la génération d'un signal de réglage suite au toucher de la surface de capteur (6 - 6C) ou à l'approche de la surface de capteur (6 - 6C) par la génération des vibrations de bruits de structure et par la transmission des vibrations de bruits de structure sur une surface d'affichage du dispositif d'affichage (5) et / ou sur la surface de capteur (6 - 6C).

2. Instrument de traitement (1) médical ou dentaire tenant dans une main selon la revendication 1, **caractérisé en ce que**
la surface d'affichage du dispositif d'affichage (5) est séparée de la surface de capteur (6 - 6C) et entoure celle-ci.

3. Instrument de traitement (1) médical ou dentaire tenant dans une main selon la revendication 1 ou 2, **caractérisé en ce que**
le générateur de vibrations (7) du dispositif d'affichage (5) comporte un élément piézoélectrique.

4. Instrument de traitement (1) médical ou dentaire tenant dans une main selon l'une quelconque des revendications précédentes, **caractérisé en ce que**
le générateur de vibrations (7) du dispositif d'affichage (5) comporte un moteur à balourds.

5. Instrument de traitement (1) médical ou dentaire tenant dans une main selon l'une quelconque des revendications précédentes, **caractérisé en ce que**
le dispositif d'affichage (5) pour générer des vibrations est opérationnellement connecté avec la surface d'affichage ou avec la surface de capteur (6 - 6C) par l'intermédiaire d'un composant du dispositif d'affichage (5) et / ou par l'intermédiaire d'au moins un composant de l'instrument de traitement (1), pour transmettre des vibrations sur la surface d'affichage ou sur la surface de capteur (6 - 6C).

6. Instrument de traitement (1) médical ou dentaire tenant dans une main selon l'une quelconque des revendications précédentes, **caractérisé en ce que**
le dispositif d'affichage (5) pour la génération de vibrations génère des vibrations acoustiquement perceptibles par un utilisateur.

7. Instrument de traitement (1) médical ou dentaire tenant dans une main selon l'une quelconque des revendications précédentes, **caractérisé par**
une carte de circuit imprimé (11) placée à l'intérieur de l'instrument de traitement (1), sur laquelle est placée au moins une partie du dispositif d'affichage (5).

8. Instrument de traitement (1) médical ou dentaire tenant dans une main selon la revendication 7, **caractérisé en ce que**
sur la carte de circuit imprimé (11) est placée additionnellement au moins une partie de l'élément de réglage (3), notamment au moins une partie du capteur (4) capacitif.

9. Instrument de traitement (1) médical ou dentaire tenant dans une main selon l'une quelconque des revendications précédentes, **caractérisé par**
une électronique de commande (12) placée dans l'instrument de traitement (1), qui est connectée avec le capteur (4) capacitif et avec le dispositif d'affichage (5) et qui est conçue pour réceptionner le signal de réglage de la part du capteur (4) capacitif et pour commander le dispositif d'affichage (5) sur la base du signal de réglage réceptionné, pour afficher un toucher de la surface de capteur (6 - 6C) ou une approche de la surface de capteur (6 - 6C).

10. Instrument de traitement (1) médical ou dentaire tenant dans une main selon la revendication 9, **caractérisé en ce que**
l'électronique de commande (12) est connectée par ailleurs avec un actionneur (13) et est prévue pour commander l'actionneur (13) sur la base du signal de réglage réceptionné de la part du capteur (4) capacitif.

11. Instrument de traitement (1) médical ou dentaire tenant dans une main selon l'une quelconque des revendications précédentes, **caractérisé par**
un dispositif de transmission (15), qui est conçu pour transmettre le signal de réglage ou un signal de commande déclenché par le signal de réglage à un contrôleur (16) externe.

12. Instrument de traitement (1) médical ou dentaire tenant dans une main selon l'une quelconque des revendications précédentes, **caractérisé en ce que**
le dispositif d'affichage (5) comporte un marquage (17) visible et / ou tangible par l'utilisateur sur la surface de la gaine extérieure (2) .

13. Instrument de traitement (1) médical ou dentaire tenant dans une main selon la revendication 12, **caractérisé en ce que**
le marquage (17) comporte au moins l'un des éléments suivants: un marquage en couleur; un marquage haptiquement perceptible par un utilisateur, qui pour la perception haptique comporte au moins un creux et / ou un relief.

14. Instrument de traitement (1) médical ou dentaire tenant dans une main selon la revendication 12 ou 13, **caractérisé en ce que**
le marquage (17) est fixé ou appliqué sur la gaine extérieure (2) et / ou sur la surface de capteur (6 - 6C) ou est créé sur ou par la gaine extérieure (2) et / ou la surface de capteur (6 - 6C)

15. Instrument de traitement (1) médical ou dentaire tenant dans une main selon l'une quelconque des revendications précédentes, **caractérisé en ce que**
l'élément de commande (3) et le dispositif d'affichage (5) sont prévus sur un segment de raccord de flexible (1C) de l'instrument de traitement (1) destiné à être connecté sur une source de fluide, une source d'énergie ou un dispositif de réglage (16).
